**Europäisches Patentamt**

**European Patent Office**

**Office européen des brevets**

(19)

(11) Veröffentlichungsnummer: **0 132 736**
**B1**

(12) **EUROPÄISCHE PATENTSCHRIFT**

(45) Veröffentlichungstag der Patentschrift:
**07.10.87**

(51) Int. Cl.⁴: **C 07 C 85/18**, C 07 C 85/02

(21) Anmeldenummer: **84108308.2**

(22) Anmeldetag: **14.07.84**

(54) **Verfahren zur Herstellung von Aminen.**

(30) Priorität: **23.07.83 DE 3326579**

(43) Veröffentlichungstag der Anmeldung:
**13.02.85 Patentblatt 85/7**

(45) Bekanntmachung des Hinweises auf die Patenterteilung:
**07.10.87 Patentblatt 87/41**

(84) Benannte Vertragsstaaten:
**AT BE CH DE FR GB IT LI NL**

(56) Entgegenhaltungen:
**EP - A - 0 039 918**
**EP - A - 0 051 741**
**EP - A - 0 077 016**

(73) Patentinhaber: **BASF Aktiengesellschaft,
Carl-Bosch-Strasse 38, D-6700 Ludwigshafen (DE)**

(72) Erfinder: **Taglieber, Volker, Dr., Franz-Liszt-Strasse 15,
D-6904 Eppelheim (DE)**
Erfinder: **Hoelderich, Wolfgang, Dr., Mannheimer
Strasse 18c, D-6710 Frankenthal (DE)**
Erfinder: **Kummer, Rudolf, Dr., Kreuzstrasse 6,
D-6710 Frankenthal (DE)**
Erfinder: **Mross, Wolf Dieter, Dr.,
Anselm-Feuerbach-Strasse 21, D-6710 Frankenthal (DE)**
Erfinder: **Saladin Guenter, Muenchbuschweg 73,
D-6700 Ludwigshafen (DE)**

ACTORUM AG

## Beschreibung

Die Erfindung betrifft ein Verfahren zur Herstellung von Aminen durch Anlagerung von Ammoniak oder analog reagierenen Aminen an Olefine.

Bei der Addition von Ammoniak oder analog reagierenden Aminen an Olefine handelt es sich um eine seit langem bekannte und vielfach beschriebene Reaktion. Eine technische Realisierung ist bislang nicht erfolgt, da nach den bekannten Verfahren nur ungenügende Selektivitäten und/oder Katalysator-Standzeiten erreicht werden, wie beispielsweise nach US-PS 2 623 061, US-PS 2 422 631, US-PS 2 501 556 und US-PS 3 412 158. Auch das in US-PS 4 307 250 beschriebene Verfahren eignet sich nicht für die Durchführung im technischen Massstab. Die als Katalysator verwendeten Alumino-Zeolithe des Y-Typs begünstigen starke Polymerbildung und nachfolgende Verkokung, die den Katalysator schnell desaktiviert.

Es wurde nun gefunden, dass man hohe Standzeiten und hohe Selektivität der verwendeten Katalysatoren für die erstellung von Aminen aus Olefinen und Ammoniak oder primären und/oder sekundären Aminen bei Temperaturen zwischen 80 und 400 °C und bei Drücken zwischen 40 und 700 bar erzielt, wenn man Olefine und Ammoniak, primäre und/oder sekundäre Amine oder Gemische derselben in Gegenwart eines Zeolithkatalysators vom Pentasil-Typ umsetzt, das erhaltene Amin abtrennt und die nichtumgesetzten Einsatzstoffe zurückführt.

Weiterhin zeichnet sich das erfindungsgemässe Verfahren dadurch aus, dass bereits mit niedrigem Ammoniak- beziehungsweise Aminüberschuss eine hohe Selektivität an gewünschtem Reaktionsprodukt erzielt und die Dimerisierung und/oder Oligomerisierung des eingesetzten Olefins vermieden wird.

Besonders gute Ergebnisse erzielt man, wenn man als Katalysator Aluminosilikat-Zeolithe des Pentasil-Typs mit einem $SiO_2/Al_2O_3 \geq 10$ verwendet.

Eine Ausführungsform des Verfahrens besteht darin, dass man Ammoniak und/oder die Amine zusammen mit Olefinen im molaren Verhältnis von 1:1 bis 5:1 gemischt einem Festbett- oder Wirbelbettreaktor zuführt und bei einem Druck von 40 bis 700 bar, insbesondere 200 bis 300 bar, und einer Temperatur von 80 bis 400 °C, insbesondere von 250 bis 350 °C, in der Gasphase oder im überkritischen Zustand umsetzt. Eine andere Ausführungsform besteht darin, dass die Reaktion in Flüssigphase bei einem Druck von 40 bis 80 bar und einer Temperatur von 60 °C bis 120 °C in einem Rührkessel, Fest-/Flüssigkeit-Fliessbett oder einem Strömungsrohr durchgeführt wird.

Aus dem Reaktionsaustrag wird das gewünschte Produkt mit Hilfe bekannter Methoden, beispielsweise Destillation oder Extraktion, erhalten und nötigenfalls mittels weiterer Trennoperationen auf die gewünschte Reinheit gebracht. Die nichtumgesetzten Eingangsstoffe werden in den Reaktor zurückgeführt.

Man kann einfach oder mehrfach ungesättigte Olefine mit 2 bis 10 C-Atomen bzw. deren Mischungen als Ausgangsstoffe verwenden. Wegen der geringen ausgeprägten Polymerisiationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstigt das Additionsprodukt, doch stellt im allgemeinen aus technischen und wirtschaftlichen Gründen der Druckbereich bis 300 bar das Optimum dar. Die Selektivität der Reaktion wird – Neben Grössen wie Ammoniak-/Amin-Überschuss und Katalysator – in hohem Mass durch die Temperatur beeinflusst. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Die Lage des Temperaturoptimums bezüglich Umsatz und Selektivität ist von der Konstitution des Olefins, des eingesetzten Amins und des Katalysators abhängig und liegt meist im Bereich von 250 bis 350 °C. Die Verweilzeit ist abhängig von den Einsatzstoffen und liegt zweckmässigerweise im Bereich zwischen Bruchteilen einer Sekunde und wenigen Minuten.

Als Katalysatoren für die Aminierung von Olefinen verwendet man Zeolithe des Pentasiltyps. Die Zeolithe können unterschiedliche chemische Zusammensetzung aufweisen, z.B. Alumino-, Eisen-, Gallium-, Chrom-, Arsen- und Wismutsilikatzeolithe oder deren Gemische, sowie Alumino-, Gallium- und Eisengermanatzeolithe oder deren Gemische.

Insbesondere eignen sich die Aluminosilikat- oder Eisensilikatzeolithe. Geeignete Aluminosilikat- oder Eisensilikatzeolithe erhält man z.B. aus einer Aluminiumverbindung, vorzugsweise $Al(OH)_3$ oder $Al_2(SO_4)_3$ bzw. Eisenverbindung, vorzugsweise $Fe_2(SO_4)_3$, und einer Siliciumverbindung, vorzugsweise hochdisperses Siliciumdioxid, in wässriger Aminlösung, insbesondere in 1,6-Hexandiamin- oder 1,3-Propandiamin-oder Triethylentetramin-Lösung mit und ohne Alkali-oder Erdalkalizusatz bei 100 bis 220 °C unter autogenem Druck. Die synthetisisierten Aluminosilikatzeolithe haben je nach Wahl der Einsatzstoffmengen ein $SiO_2/Al_2O_3$-Verhältnis von 10 bis 40 000.

Geeignete Aluminosilikat- und Eisensilikatzeolithe kann man auch in etherischem Medium wie Diethylenglykoldimethylether, in alkoholischem Medium wie Methanol bzw. 1,4-Butandiol aber auch in Wasser synthetisieren.

Die so hergestellten Aluminosilikat- und Eisensilikatzeolithe kann man nach ihrer Isolierung, Trocknung bei 100 bis 160 °C, vorzugsweise bei etwa 110 °C, und Calcinierung bei 450 bis 550 °C, vorzugsweise bei etwa 500 °C, mit einem Bindemittel im Verhältnis 90:10 bis 40:60 Gew.-% zu Strängen oder Tabletten verformen. Als Bindemittel eignen sich verschiedene Aluminiumoxide, bevorzugt Boehmit, amorphe Aluminosilikate mit einem $SiO_2/Al_2O_3$-Verhältnis von 25:75 bis 95:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Presslinge zweckmässig bei 110 °C/16 h getrocknet und bei 500 °C/16 h calciniert.

Eine besondere Ausführungsform besteht darin, dass der isolierte Aluminosilikat- oder Eisensilikatzeolith direkt nach der Trocknung verformt wird und erstmals nach der Verformung einer Calcination unterworfen wird.

Aus dem zu Strängen verformten Katalysator kann man durch Mahlen und Sieben Wirbelgut in der Grösse von 0,1 bis 0,8 mm erhalten.

Nach Desaktivierung der zeolithischen Katalysatoren durch Koksabscheidung während der erfindungsgemässen Umsetzung kann man die Katalysatoren durch Abbrennen der Koksablagerung mit Luft oder mit einem Luft/$N_2$-Gemisch bei 400 bis 550 °C, bevorzugt bei etwa 500 °C, in einfacher Weise regenerieren. Sie erhalten dadurch ihre Anfangsaktivität zurück.

Zur Erhöhung der Selektivität, der Standzeit und der Anzahl der möglichen Regenerierungen kann man verschiedene Modifizierungen an den zeolithischen Katalysatoren vornehmen.

Eine Modifizierung der Katalysatoren besteht darin, dass man die unverformten oder die verformten Zeolithe mit Alkalimetallen wie Na und K, Erdalkalimetallen wie Ca, Mg, Erdmetallen wie Tl, Übergangsmetallen wie Mn, Fe, Mo, Cu, Zn und seltenen Erdmetallen wie La, Ce ionenaustauschen bzw. dotieren kann.

Eine vorteilhafte Ausführungsform besteht darin, dass man die verformten Pentasilzeolithe in einem Strömungsrohr vorlegt und bei 20 bis 100 °C, z.B. ein Halogenid oder ein Nitrat der oben beschriebenen Metalle überleitet. Ein derartiger Ionenaustausch kann z.B. an der Wasserstoff-, Ammonium- und Alkaliform der Zeolithe vorgenommen werden.

Eine weitere Möglichkeit der Metallaufbringung auf die Zeolithe besteht darin, dass man das zeolithische Material, z.B. mit einem Halogenid, einem Nitrat oder einem Oxid der oben beschriebenen Metalle in wässriger oder alkoholischer Lösung imprägniert.

Sowohl an einen Ionenaustausch als auch an eine Imprägnierung kann man eine Trocknung, wahlweise eine abermalige Calcination anschliessen. Bei metalldotierten Zeolithen kann eine Nachbehandlung mit Wasserstoff und/oder mit Wasserdampf günstig sein.

Eine weitere Möglichkeit der Modifizierung besteht darin, dass man das zeolithische Material – verformt oder unverformt – einer Behandlung mit Säuren, wie Salzsäure-, Flusssäure- und Phosphorsäure unterwirft.

Eine besondere Ausführungsform besteht darin, dass man das zeolithische Pulver vor seiner Verformung mit Flusssäure 0,001 n bis 2 n, bevorzugt 0,05 bis 0,5 n 1 bis 3 Stunden unter Rückfluss behandelt. Nach Abfiltrieren und Auswaschen wird bei 100 bis 160 °C getrocknet und bei 400 bis 550 °C calciniert. Eine weitere besondere Ausführungsform liegt in einer HCl-Behandlung der Zeolithe nach ihrer Verformung mit Bindemittel. Hierbei wird der Zeolith 1 bis 3 Stunden zwischen 60 und 80 °C mit einer 3 bis 25%igen, insbesondere mit einer 12 bis 20%igen Salzsäure behandelt, anschliessend ausgewaschen, bei 100 bis 160 °C getrocknet und bei 400 bis 550 °C calciniert.

Eine andere Möglichkeit der Modifizierung ist gegeben durch einen Austausch mit Ammonsalzen, z.B. mit $NH_4Cl$ oder mit Mono- oder Di- oder Polyaminen. Hierbei wird der in der H-Form oder einer anderen Ammoniumform vorliegende, mit Bindemittel verformte Zeolith bei 60 bis 80 °C mit 10 bis 25%iger, bevorzugt 20%iger $NH_4Cl$-Lösung 2 h kontinuierlich in gewichtsmässiger Zeolith/Ammonchlorid-Lösung von 1:15 ausgetauscht und danach bei 100 bis 120 °C getrocknet.

Die Katalysatoren kann man als 2 mm bis 4 mm-Stränge, als Tabletten mit 3 bis 5 mm Durchmesser, als Wirbelgut mit einer Grösse von 0,1 bis 0,8 mm oder als Splitt mit 0,5 bis 1 mm Durchmesser für die Aminierung der Olefine einsetzen.

Die nachfolgenden Beispiele veranschaulichen die Erfindung.

Beispiel 1

Als Katalysator A wird ein Aluminosilikatzeolith des Pentasiltyps unter hydrothermalen Bedingungen bei autogenem Druck und 150 °C aus 65 g $SiO_2$ (hochdispers) und 20,3 g $Al_2(SO_4)_3 \times 18\ H_2O$ in 1 kg einer wässrigen 1,6-Hexandiamin-Lösung (Mischung 50:50 Gew.-%) in einem Rührautoklaven synthetisiert. Nach Abfiltrieren und Auswaschen wird das kristalline Reaktionsprodukt bei 110 °C/24 h getrocknet und bei 500 °C/24 h calciniert. Dieser Aluminosilikatzeolith enthält 91,6 Gew.-% $SiO_2$ und 4,6 Gew.-% $Al_2O_3$. Dieses Produkt wird mit Boehmit im Gewichtsverhältnis 60:40 zu 2 mm-Strängen verformt und bei 110 °C/16 h getrocknet und bei 500 °C/24 h calciniert.

Als Katalysator B werden 50 g des Katalysators A mit 9,39 g $Zn(NO_3)_2 \times 6\ H_2O$ – in 20 g $H_2O$ gelöst – getränkt. Nach dem Tränken wird bei 130 °C/2 h getrocknet und bei 540 °C/2 h calciniert.

In einen 0,3 l Rührautoklaven werden jeweils 10 ml der oben beschriebenen Katalysatoren A oder B gegeben und nach dem Verschliessen die Olefine und Ammoniak bzw. Amine aufgedrückt. Die Menge an Einsatzprodukt wird so bemessen, dass bei der gewählten Reaktionstemperatur der gewünschte Druck als Eigendruck der Reaktionspartner erreicht wird. Das molare Verhältnis von Ammoniak/Amin zu Olefin wird von 1:1 bis 5:1 variiert, die Reaktionsdauer auf 30 min. festgelegt.

Der Austrag wurde getrennt nach Flüssig- und Gasphase gaschromatographisch untersucht. Die in Tabelle 1 aufgeführten Umsätze beziehen sich immer auf das Olefin; die angegebenen Selektivitäten beziehen sich auf die Hauptprodukte: Ethylamin aus Etyhlen, Isopropylamin aus Propylen, Isobutylamin aus Buten-1, tertiär-Butylamin aus Isobuten, Isopentylamin aus Isobuten mit Methylamin und 1-Amino-4-(1-aminoethyl)cyclohexan aus 4-Vinyl-1-cyclohexen.

Tabelle 1

| Olefin | Amin | Amin:Olefin [mol/mol] | Katalysator | Temperatur [°C] | Druck [bar] | Umsatz [%] | Selektivität [%] |
|---|---|---|---|---|---|---|---|
| Ethylen | $NH_3$ | 2:1 | A | 350 | 300 | 3,2 | 94,7 |
| Ethylen | $NH_3$ | 2:1 | A | 380 | 305 | 6,5 | 93,3 |
| Ethylen | $NH_3$ | 2:1 | B | 350 | 290 | 4,8 | 95,7 |
| Propylen | $NH_3$ | 2:1 | A | 330 | 285 | 7,3 | 97,3 |
| Propylen | $NH_3$ | 2:1 | A | 350 | 295 | 10,4 | 96,5 |
| Propylen | $NH_3$ | 2:1 | B | 330 | 300 | 8,7 | 97,6 |
| Buten-1- | $NH_3$ | 1,5:1 | A | 300 | 290 | 9,1 | 96,1 |
| Isobuten | $NH_3$ | 1,5:1 | A | 320 | 295 | 10,9 | 97,9 |
| Isobuten | $NH_3$ | 1,5:1 | A | 330 | 590 | 12,1 | 97,4 |
| Isobuten | $NH_3$ | 1,5:1 | B | 330 | 285 | 10,8 | 97,9 |
| Isobuten | $NH_3$ | 1,5:1 | B | 330 | 540 | 11,8 | 96,8 |
| Isobuten | | 2:1 | A | 330 | 240 | 7,7 | 91,8 |
| 4-Vinyl-1-cyclohexen | $NH_3$ | 4:1 | A | 330 | 275 | 6,8 | 85,7 |

Beispiel 2

Für eine kontinuierliche Herstellung wird ein Hochdruck-Reaktor mit 2 m Länge und 24 mm Innendurchmesser eingesetzt, der mittels einer Alublockheizung beheizt und mit dreifacher Innentemperaturmessung sowie mit einer Druckhaltung ausgestattet ist. Es werden jeweils 60 ml Katalysator eingebaut und der obere Teil des Reaktorrohres mit Porzellanringen gefüllt. Der Zulauf von Olefin und Amin erfolgt von oben.

Die Analyse der Reaktorausträge wird gaschromatographisch und z.T. zusätzlich destillativ durchgeführt.

Die Ergebnisse mit Katalysator A sind in Tabelle 2 wiedergegeben.

Tabelle 2

| Olefin | Amin | Amin: Olefin [mol/mol] | Katalysator | Katalysator-Belastung [kg/l K.h] | Temperatur [°C] | Druck [bar] | Umsatz [%] | Selekt. [%] | RZA [kg/l K.h] |
|---|---|---|---|---|---|---|---|---|---|
| Isobuten | $NH_3$ | 1,5:1 | A | 9 | 320 | 300 | 6,6 | 98,7 | 0,33 |
| Isobuten | $NH_3$ | 1,5:1 | A | 9 | 350 | 300 | 8,4 | 97,6 | 0,62 |

RZA = Raum-Zeit-Ausbeute tertiär-Butylamin

Beispiel 3

Katalysator C wird aus einem Eisensilikatzeolith durch Verformung zu Strängen mit Boehmit im Gewichtsverhältnis 60:40 und anschliessender Calcination bei 500 °C/16 h hergestellt. Der Eisensilikatzeolith des Pentasil-Typs wird unter hydrothermalen Bedingungen bei autogenem Druck und 165 °C aus 273 g Wasserglas, gelöst in 253 g einer wässrigen 1,6-Hexandiamin-Lösung (Mischung 50:50 Gew.-%), und 31 g Eisensulfat, gelöst in 21 g 96%iger Schwefelsäure und 425 g Wasser, in einem Rührautoklaven während 4 Tagen synthetisiert, danach abfiltriert, ausgewaschen, bei 110 °C/24 h getrocknet und bei 500 °C/24 h calciniert. Dieser Eisensilikatzeolith hat ein $SiO_2/Fe_2O_3$-Verhältnis von 17,7 und einen $Na_2O$-Gehalt von 0,62 Gew.-%.

Katalysator D wird erhalten, indem man 50 g des Katalysators A mit 7,9 g $Mn(NO_3)_2 \times 4 H_2O$ – in 20 g $H_2O$ gelöst – imprägniert, bei 130 °C/2 h getrocknet und bei 540 °C/2 h calciniert.

In einem Rohrreaktor (6 mm Innendurchmesser) wird unter isothermen Bedingungen bei 300 °C und einem Druck von 300 bar ein Gemisch aus Isobuten und Ammoniak im molaren Verhältnis von 1:1,3 an den Katalysatoren A, B, C und D diskontinuierlich umgesetzt. Die Reaktionsprodukte werden laufend im Gaschromatographen analysiert. Die Versuchsergebnisse sind in Tabelle 3 zusammengefasst.

Tabelle 3

| Katalysator | A | B | C | C | D |
|---|---|---|---|---|---|
| Temperatur [ °C] | 300 | 300 | 300 | 300 | 300 |
| Druck [bar] | 300 | 300 | 300 | 300 | 300 |
| GHSV 1 Edukt g Kat. h. | 10 | 5,0 | 11 | 9,5 | 10 |
| Umsatz von Isobuten [%] | 11,1 | 12,1 | 11,2 | 9,5 | 9,0 |
| Selektivität tert.-Butylamin [%] | 93,6 | 95,2 | 94,9 | 96,0 | 90,1 |

## Patentansprüche

1. Verfahren zur Herstellung von Aminen aus Olefinen und Ammoniak oder primären oder sekundären Aminen bei Temperaturen zwischen 80 und 400 °C, bei Drücken zwischen 40 und 700 bar in Gegenwart eines zeolithischen Katalysators, dadurch gekennzeichnet, dass man Olefin und Ammoniak oder primäres oder sekundäres Amin oder Gemische derselben in Gegenwart eines Zeolithkatalysators vom Pentasil-Typ umsetzt, das erhaltene Amin abtrennt und die nichtumgesetzten Einsatzstoffe zurückführt.

2. Verfahren nach Anspruch 1, dadurch gekennzeichnet, dass man als Katalysator Aluminosilikatzeolithe des Pentasil-Typs mit einem $SiO_2/Al_2O_3 \geqq 10$ verwendet.

3. Verfahren nach Anspruch 1, dadurch gekennzeichnet, dass man als Katalysator Eisensilikatzeolithe des Pentasil-Typs verwendet.

4. Verfahren nach Anspruch 1 bis 3, dadurch gekennzeichnet, dass der Katalysator mit einem Bindemittel verformt und anschliessend calciniert wird.

5. Verfahren nach Anspruch 1 bis 4, dadurch gekennzeichnet, dass der Katalysator einer Säurebehandlung unterworfen wird.

6. Verfahren nach Anspruch 1 bis 5, dadurch gekennzeichnet, dass der Katalysator mit Übergangsmetallen dotiert wird.

7. Verfahren nach Anspruch 1 bis 5, dadurch gekennzeichnet, dass der Katalysator mit seltenen Erden dotiert wird.

8. Verfahren nach Anspruch 1 bis 5, dadurch gekennzeichnet, dass der Katalysator mit Alkali-, Erdalkali- und/oder Erdmetallen dotiert wird.

9. Verfahren nach Anspruch 1 bis 5, dadurch gekennzeichnet, dass der Katalysator nach Behandlung mit Ammonsalzen in seiner Ammoniumform eingesetzt wird.

## Revendications

1. Procédé de préparation d'amines à partir d'oléfines et d'ammoniac ou d'amines primaires ou secondaires, à des températures qui varient de 80 à 400 °C, sous des pressions qui fluctuent de 40 à 700 bars, en présence d'un catalyseur du type des zéolites, caractérisé en ce que l'on fait réagir l'oléfine et l'ammoniac ou l'amine primaire ou secondaire ou des mélanges de ces composés, en présence d'une zélithe servant de catalyseur du type pentasile, on sépare l'amine obtenue et on recycle les matières de départ non entrées en réaction.

2. Procédé suivant la revendication 1, caractérisé en ce qu'à titre de catalyseur, on utilise des aluminosilicatozélites du type pentasile avec un rapport $SiO_2/Al_2O_3 \geqq 10$.

3. Procédé suivant la revendication 1, caracatérisé en ce qu'à titre de catalyseur, on utilise des sidéro-silicato-zéolites du type pentasile.

4. Procédé suivant les revendications 1 à 3, caractérisé en ce quel'on transforme le catalyseur avec un liant et on le calcine ensuite.

5. Procédé suivant les revendications 1 à 4, caractérisé en ce que l'on soumet le catalyseur à un traitement à l'acide.

6. Procédé suivant les revendications 1 à 5, caractérisé en ce que l'on dope le catalyseur avec des métaux de transition.

7. Procédé suivant les revendications 1 à 5, caractérisé en ce que l'on dope le catalyseur avec des terres rares.

8. Procédé suivant les revendications 1 à 5, caractérisé en ce que l'on dope le catalyseur avec des métaux alcalins, alcalino-terreux et/ou terreux.

9. Procédé suivant les revendications 1 à 5, caractérisé en ce que l'on utilise le catalyseur après son traitement par des sels ammoniques sous sa forme ammonium.

## Claims

1. A process for the preparation of an amine from an olefin and ammonia or a primary or secondary amine at from 80 to 400 °C and under from 40 to 700 bar in the presence of a zeolite catalyst, wherein an olefin is reacted with ammonia or a primary or secondary amine, or a mixture of these, in the presence of a zeolite catalyst of the pentasil type, the resulting amine is isolated, and the unreacted starting materials are recycled.

2. A process as claimed in claim 1, wherein the catalyst used is an aluminosilicate zeolite of the pentasil type having an $SiO_2/Al_2O_3$ ratio of $\geqq 10$.

3. A process as claimed in claim 1, wherein the catalyst used is an iron silicate zeolite of the pentasil type.

4. A process as claimed in claims 1 to 3, wherein the catalyst is molded together with a binder and then calcined.

5. A process as claimed in claims 1 to 4, wherein the catalyst is treated with an acid.

6. A process as claimed in claims 1 to 5, wherein the catalyst is doped with a transition metal.

7. A process as claimed in claims 1 to 5, wherein the catalyst is doped with a rare earth.

8. A process as claimed in claims 1 to 5, wherein the catalyst is doped with an alkali metal, an alkaline earth metal and/or an earth metal.

9. A process as claimed in claims 1 to 5, wherein the catalyst is treated with an ammonium salt and then used in its ammonium form.